(19) Europäisches Patentamt
European Patent Office
Office européen des brevets

(11) Veröffentlichungsnummer: **0 164 590**
**B1**

(12) # EUROPÄISCHE PATENTSCHRIFT

(45) Veröffentlichungstag der Patentschrift: **31.08.88**

(51) Int. Cl.4: **C 07 C 59/90**, C 07 C 69/738

(21) Anmeldenummer: **85105831.3**

(22) Anmeldetag: **28.05.82**

(54) **Neue 4-(3'-Trifluormethyl-phenoxy)-benzoyl-essigsäure-Derivate.**

(30) Priorität: **03.06.81 CH 3630/81**

(43) Veröffentlichungstag der Anmeldung: **18.12.85 Patentblatt 85/51**

(45) Bekanntmachung des Hinweises auf die Patenterteilung: **31.08.88 Patentblatt 88/35**

(84) Benannte Vertragsstaaten: **AT BE CH DE FR GB IT LI NL SE**

(56) Entgegenhaltungen: **GB-A-1 382 267**

(73) Patentinhaber: **CIBA- GEIGY AG, Klybeckstrasse 141, CH- 4002 Basel (CH)**

(72) Erfinder: **Tobler, Hans, Dr., Baselmattweg 157, CH- 4123 Allschwil (CH)**
Erfinder: **Föry, Werner, Dr., Benkenstrasse 65, CH- 4054 Basel (CH)**

**EP 0 164 590 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue 4-(3'-Trifluormethylphenoxy)-benzoylessigsäure-Derivate der Formel II,

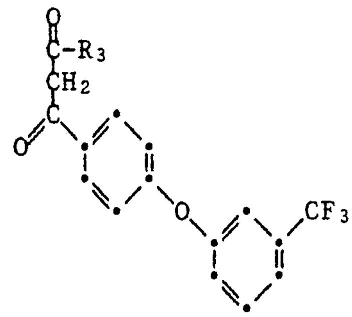

(II),

worin $R_3$ $C_1$-$C_4$-Alkoxy, Allyloxy, Propargyloxy, 2,2,2-Trichloräthoxy, 2,2,2-Trifluoräthoxy, 2-Chloräthoxy, $C_2$-$C_5$-Alkoxyalkoxy, Hydroxy oder Salze dieser Carbonsäuren bedeutet.

Die Verbindungen der Formel II sind wertvolle Zwischenprodukte zur Herstellung von neuen, in der europäischen Patentschrift EP-B-0 069 055 beschriebenen, herbizid wirksamen Pyronen der Formel

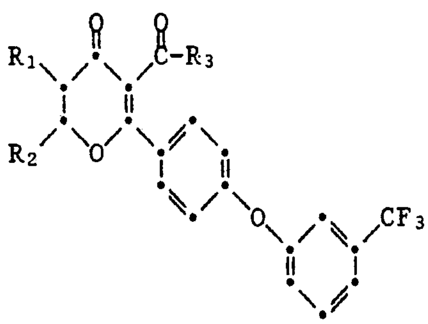

(I) ,

worin

$R_1$, und $R_2$ unabhängig voneinander eine $C_1$-$C_4$-Alkylgruppe oder eine der beiden Substituenten auch Wasserstoff, oder $R_1$, und $R_2$ gemeinsam eine $C_2$-$C_6$-Alkylenbrücke und

$R_3$ die unter Formel II angegebene Bedeutung haben.

Unter $C_1$-$C_4$-Alkyl als Substituent oder Teil eines Substituenten ist n-Propyl, Isopropyl, n-Butyl, sek.-Butyl, tert.-Butyl, Isobutyl und insbesondere Methyl und Äthyl zu verstehen.

Als Salze kommen insbesondere Metallsalze und Salze mit quaternären Ammoniumbasen oder organischen Stickstoffbasen in Betracht. Zur Salzbildung geeignete Metalle sind beispielsweise Erdalkalimetalle, wie Magnesium oder Calcium, vor allem aber die Alkylimetalle, wie Lithium, Kalium oder Natrium. Ferner sind als Salzbildner auch übergangsmetalle wie beispielsweise Eisen, Nickel, Kobalt, Kupfer, Zink, Chrom oder Mangan geeignet.

Beispiele für queternäre Ammoniumbasen sind das Ammoniumkation, Tetraalkylammoniumkationen, in denen die Alkylreste unabhängig voneinander geradkettige oder verzweigte $C_1$-$C_6$-Alkylgruppen sind, wie das Tetramethylammoniumkation, das Tetraäthylammoniumkation oder das Trimethyläthylammoniumkation sowie weiterhin das Trimethylbenzylammoniumkation, das Triäthylbenzylammoniumkation, das Trimethyl-2-hydroxyäthylammoniumkation und das Trimethyl-2-chloräthylammoniumkation.

Beispiele für zur Salzbildung geeignete organische Stickstoffbasen sind primäre, sekundäre und tertiäre, aliphatische und aromatische, gegebenenfalls am Kohlenwasserstoffrest hydroxylierte Amine, wie Methylamin, Äthylamin, Propylamin, Isopropylamin, die vier isomeren Butylamine, Dimethylamin, Diäthylamin, Di-n-propylamin, Di-isopropylamin, Di-n-butylamin, Pyrrolidin, Piperidin, Morpholin, Trimethylamin, Triäthylamin, Tri-n-propylamin, Chinuclidin, Pyridin, Chinolin, Isochinolin sowie Methanolamin, Äthanolamin, Propanolamin, Dimethanolamin, Diäthanolamin oder Triäthanolamin.

Die Herstellung von Verbindungen der Formel I kann analog dem in Bull. Soc. Chim. de France (1968), 288 - 298, beschriebenen Verfahren erfolgen, indem man

a) eine Verbindung der Formel II

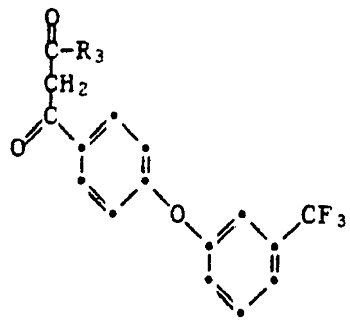

(II),

worin $R_3$ die vorstehend unter Formel II angegebene Bedeutung hat, in einem inerten Lösungsmittel mit einer Verbindung der Formel III

$Mg(OR')_2$ (III),

worin R' eine geradkettige oder verzweigte $C_1$-$C_4$-Alkylgruppe bedeutet, umsetzt und

b) das erhaltene Reaktionsprodukt in einem inerten Lösungsmittel mit einer Verbindung der Formel V

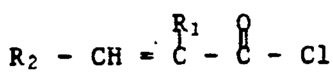

(V),

worin $R_1$ und $R_2$ die unter Formel I angegebenen Bedeutungen haben, umsetzt und

c) das erhaltene Produkt mit einer alkoholischen Lösung eine starken Säure cyclisiert und, wenn $R_3$ in der Formel I für Hydroxy steht, den erhaltenen Ester verseift, oder gegebenenfalls in dem erhaltenen Ester der Formel I den Rest $R_3$ durch Umesterung in einen anderen, unter Formel I definierten Rest $R_3$ überführt.

Die unter a) und b) beschriebenen Umsetzungen werden jeweils in einem inerten Lösungsmittel durchgeführt. Als geeignete Lösungsmittel kommen beispielsweise Benzol, Toluol, Xylol, Äther, Tetrahydrofuran oder Dioxan in Betracht.

Die Umsetzung einer Verbindung der Formel II mit einer Verbindung der Formel III erfolgt zweckmässigerweise bei einer Temperatur im Bereich von 0 bis 150°C, die Umsetzung des unter a) erhaltenen Reaktionsproduktes mit einer Verbindung der Formel V bei einer Temperatur im Bereich von -10°C bis Raumtemperatur.

Das in der unter a) beschriebenen Umsetzung verwendete Magnesiumalkoholat der Formel III kann in situ durch Umsetzfung von Magnesium mit einem entsprechenden Alkohol in Gegenwart von $CCl_4$ hergestellt werden.

Die unter c) beschriebene Cyclisierung lässt sich mit einer alkoholischen Lösung einer katalytischen Menge einer starken Säure am Rückfluss durchführen, beispielsweise mit einer alkoholischen Lösung von Chlorvasserstoffsäure, die auch in situ durch Umsetzung von Acetvlchlorid mit Alkohol erhältlich ist.

Die Überführung eines Restes $R_3$ in einen anderen, unter Formel I definierten Rest $R_3$ durch Umesterung kann in an sich bekannter Weise durchgeführt werden, indem man eine Verbindung der Formel I mit einem entsprechenden Alkohol in Gegenwart einer starken Säure versetzt.

Die neuen Verbindungen der Formel II sind speziell für die Herstellung der Verbindungen der Formel I entwickelt worden.

In der DE-OS-2 436 012 werden 4-Phenoxy-benzoylessigsäure und -alkylester, in denen die Phenoxygruppe unsubstituiert oder durch ein Fluor-, Chlor- oder Bromatom oder eine Trifluormethylgruppe substituiert ist, beschrieben. Die Alkylester sind durch Friedel-Crafts-Acylierung der entsprechenden Diphenyläther mit Malonesterchloriden, und die entsprechenden Säuren durch anschliessende Verseifung erhältlich.

Ausser nach dem vorstehend bereits angeführten Verfahren lassen sich Verbindungen der Formel II auch analog dem in J. Amer. Chem. Soc. 70 (1948), 3356 - 3360, beschriebenen Verfahren durch Umsetzung von Phenoxyphenylderivaten mit Malonsäuremethylesterchlorid herstellen.

Die Herstellung von Verbindungen der Formel II, in denen $R_3$ für $C_1$-$C_4$-Alkoxy steht, kann analog dem Tetrahedron 20 (1964), 1625 - 1632, beschriebenen Verfahren gemäss folgender schematischer Darstellung erfolgen:

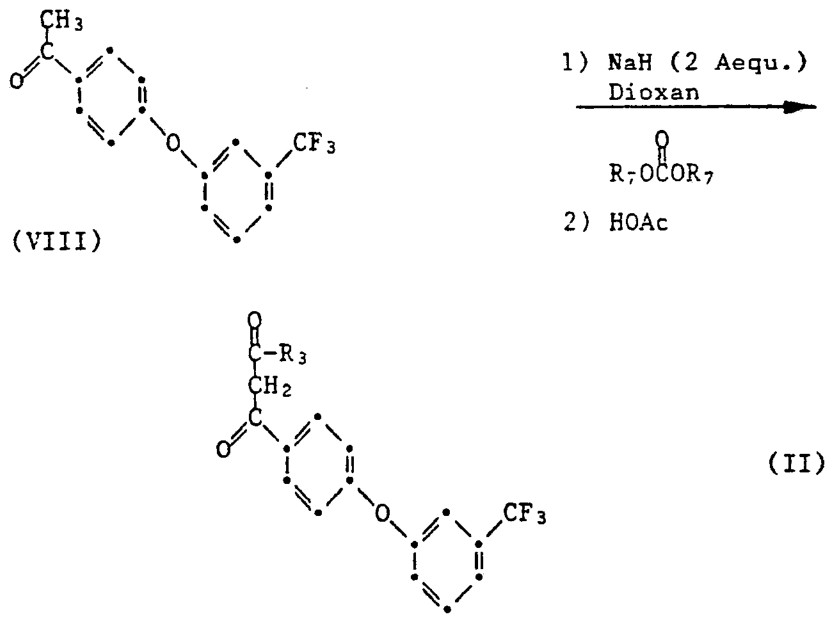


(VIII)

(II).

In den vorstehenden Formeln steht $R_7$ für $C_1$-$C_4$-Alkyl.

Die Verbindungen der Formel VIII sind bekannt oder können analog bekannten Methoden hergestellt werden (s. z. B. US-PS-4 125 729; BE-PS-639 727 [Ref. in Chem. Abst. 62 (1965), 14581h]).

**Herstellungsbeispiel für Verbindungen der Formel II.**

Beispiel 3: 4-(3'-Trifluormethyl-phenoxy)-benzoylessigsäuremethylester (Verbindung 1 in der nachstehenden Tabelle 1).

Zu einer Mischung von 29 g Nah (2 Äqu.; 55 %-ige Öldispersion, dreimal mit Toluol gewaschen) und 54 g Dimethylcarbonat in 400 ml Dioxan werden bei 85° C 84,2 g 4-(3'-Trifluormethyl-phenoxy)-acetophenon gelöst in 160 ml Dioxan, hinzugetropft, wobei sofort Wasserstoffentwicklung erfolgt. Nach dem Abklingen der Reaktion wird noch 2 Stunden bei 85° C gerührt. Unter Eiskühlung werden 100 ml Essigsäure zugetropft. Die erhaltene Paste wird mit 400 ml Äther sowie ca. 200 g Alfox 1 (Aluminiumoxid (Alumina) Woelm, Aktivitätsstufe I) neutral versetzt. Das Gemisch wird filtriert. Das Filtrat wird eingedampft, in Aceton gelöst, nacheinander mit Wasser, gesättigter $NaHCO_3$-Lösung und gesättigter NaCl-Lösung gewaschen, getrocknet und eingedampft. Das erhaltene viskose Produkt kann direkt für die Herstellung von Verbindungen der Formel I verwendet werden, oder es wird bei $10^{-2}$ Torr und 160° C destilliert oder aus Hexan umkristallisiert (Smp. 50 - 55° C). Die Ausbeute an gereinigtem Produkt beträgt ca. 65 g (65 % d. Th.).

Auf analoge Weise können auch folgende Verbindungen der Formel II hergestellt werden:

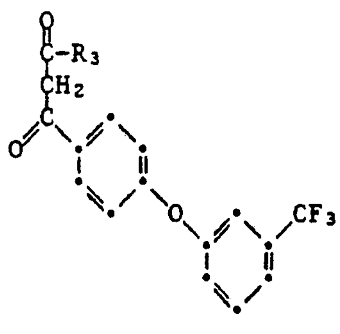

(II),

**Tabelle 1:**

| Nr. | $R_3$ | Smp °C (Konstitution) |
|---|---|---|
| 1 | $OCH_3$ | 50 - 55 |
| 2 | $OC_2H_5$ | (viskos) |
| 3 | $OC_3H_7$iso | (viskos) |
| 4 | $OC_4H_9$tert | - |
| 5 | $OC_3H_7$-n | (viskos) |
| 6 | $OC_4H_9$n | (viskos) |
| 7 | $OCH_2CH_2Cl$ | - |
| 8 | $OCH_2CH=CH_2$ | - |
| 9 | $OCH_2C\equiv CH$ | - |
| 10 | $OCH_2CH_2OCH_3$ | - |
| 11 | $OCH_2CF_3$ | - |

**Patentanspruch**

Neue 4-(3'-Trifluormethyl-phenoxy)-benzoylessigsäure-Derivate der Formel II

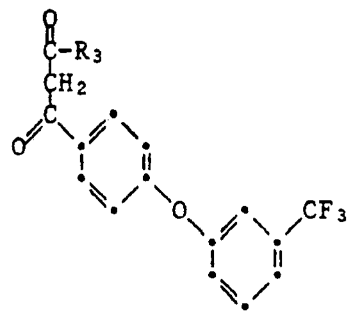

(II),

worin $R_3$ $C_1$-$C_4$-Alkoxy, Allyloxy, Propargyloxy, 2,2,2-Trichloräthoxy, 2,2,2-Trifluoräthoxy, 2-Chloräthoxy, $C_2$-$C_5$-Alkoxyalkoxy, Hydroxy oder Salze dieser Carbonsäure bedeutet.

**Claim**

Novel 4-(3'-trifluoromethyl-phenoxy)-benzoylacetic acid derivatives of the formula II

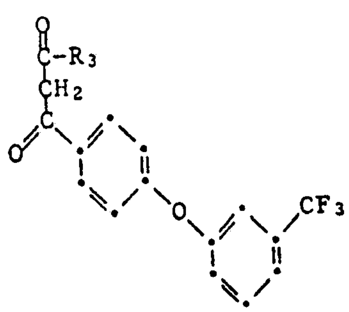

(II),

in which $R_3$ is $C_1$-$C_4$-alkoxy, allyloxy, propargyloxy, 2,2,2-trichloroethoxy 2,2,2-trifluoroethoxy, 2-chloroethoxy, $C_2$-$C_5$-alkoxyalkoxy or hydroxyl, or salts of this carboylic acid.

**Revendication**

Nouveaux dérivés de l'acide 4-(3'-trifluorométhyl-phénoxy)-benzoyl-acétique, de formule II

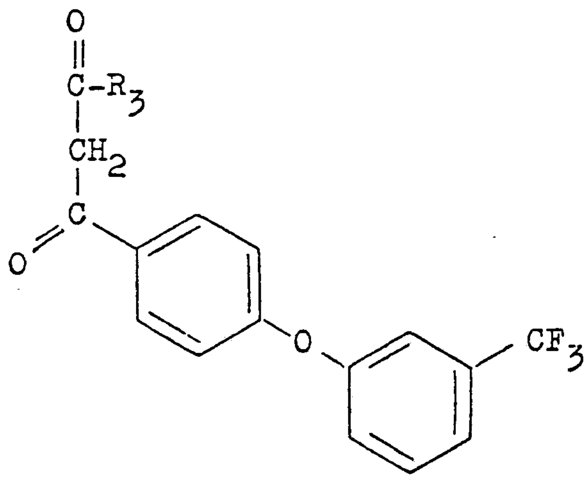

(II),

dans laquelle $R_3$ représente un groupe alcoxy en $C_1$-$C_4$, allyloxy, propargyloxy, 2,2,2-trichloréthoxy, 2,2,2-trifluoréthoxy, 2-chloréthoxy, alcoxyalcoxy en $C_2$-$C_5$, hydroxy, ou sels de cet acide carboxylique.